Europäisches Patentamt

(19) European Patent Office

Office européen des brevets

(11) Publication number: **0 287 493**
A2

# (12) EUROPEAN PATENT APPLICATION

(21) Application number: 88500038.0

(22) Date of filing: 11.04.88

(51) Int. Cl.⁴: **A 61 K 37/02**
G 01 N 33/72

(30) Priority: 14.04.87 ES 8701083

(43) Date of publication of application:
19.10.88 Bulletin 88/42

(84) Designated Contracting States:
AT BE CH DE FR GB IT LI LU NL SE

(71) Applicant: INSTITUTO LLORENTE, S.A.
General Rodrigo, 6
E-28003 Madrid (ES)

(72) Inventor: Diaz Gil, Juan José
Valdevarnés 19
E-28039-Madrid (ES)

Escartin Marin, Pedro
O'Donnell, 49
E-28009-Madrid (ES)

Garcia Canero, Rafael
Gonzalo de Córdoba, 6
E-28010-Madrid (ES)

(74) Representative: Elzaburu Marquez, Alberto et al
Sres. Elzaburu Industrial Property Attorneys Miguel
Angel, 21
E-28010 Madrid (ES)

(54) Liver growth factor.

(57) A liver growth factor, constituted by an albumin-bilirubin complex, which permits the stimulation and enhancement of liver regeneration and the diagnosis and follow-up of hepatopathies is here described.

Fig. 1. Reversed phase h.p.l.c. of tryptic digests of performic-acid-oxidized LGF (a) and purified serum albumin (b)

Samples (100 μg in 20 μl) were applied in 0.2 M-NH₄HCO₃, pH 8.0 The column was eluted at 60 ml/h: 10 mM-ammonium acetate, pH 6.5, for 8 min; a linear gradient of 0-2% acetonitrile in the acetate buffer for 10 min; a linear gradient of 2-9% acetonitrile in the same buffer for 28 min; 9% acetonitrile in acetate for 10 min; a linear gradient of 9-15% acetonitrile in the acetate buffer for 24 min; and a linear gradient of 15-35% acetonitrile in acetate buffer for 60 min. Detection of the peptides was performed by measurement of $A_{214}$.

EP 0 287 493 A2

Bundesdruckerei Berlin

**Description**

## LIVER GROWTH FACTOR

The present invention refers to a liver growth factor and its application to the diagnosis and treatment of hepatopathies. Said liver growth factor is composed of a complex of albumin and bilirubin.

BACKGROUND OF THE INVENTION

The existence of a chemical entity which provokes the regeneration of liver cells has been postulated for many years in view of the great capacity of regeneration of the liver of laboratory animals subjected to partial hepatectomy. In this regard, see the book "Regeneration of the Liver and Kidney" (Little Brown, Boston, 1971) written by Bucher, N.L.R. &; Malt, R.A. This rapid regeneration has been proved to exist in such diverse species as rat, mouse, dog, pig, monkey and even humans; nevertheless, until now, it has not been possible to determine the chemical nature of the entity responsible for said regeneration, especially in humans.

Many diverse attempts were made to purify this chemical entity from plasma, serum, hepatocellular cytosol or different extracts of animal livers after partial hepatectomy. Worthy of mention among these attempts are the following: Morley, C.G.D. et al. Biochem. Biophys. Acta 308, 260-275, 1973; Goldberg, M, et al. Metab. Res.12, 94-96, 1980; Labrecque, D.R. et al. Am. J. Physiol. 242, G281-G288, 1982; and Russell, W.E. et al. Cell. Physiol. 119, 183-192, 1984. Along this line of investigation. the authors of the invention dealt with in this application published for the first time the purification of a protein from plasma of partially hepatectomized rats, which presented the characteristics of a liver growth factor (Díaz Gil et al., Biochem. J. 235, 49-55, 1986). (This liver growth factor was first denominated HP for "hepatic promoter", to be later changed to LGF for "liver growth factor", name which agrees much better with its function). This protein, purified by means of several chromatographic steps, shows the following characteristics:

- It presented a single band in SDS-polyacrylamide-gel electrophoresis, with a molecular weight of 64,000.
- When injected into mice, whether intraperitoneally or intravenously, it produced an increase in the DNA synthesis of hepatocytes, measured by uptake of [3H]thymidine into DNA, of about 200 - 300% over controls injected with a saline solution. The maximum activity was observed with the injection of 150 ng LGF/mouse.
- Its activity was abrogated by incubation with trypsin or pepsin, but was not affected by DNase or RNase.
- Its injection into mice (150 ng LGF/mouse) increased the mitotic index of hepatocytes.
- Its action appeared to be organ-specific, acting on liver, but not on spleen, kidney, lung or brain at a dose of 150 ng LGF/mouse.
- In primary culture of rat hepatocytes, LGF produced an increase in DNA synthesis, measured by the uptake of [3H]thymidine into DNA, within the range of 1-10 ng LGF/ml.
- In this "in vitro" system, LGF likewise incremented 22Na uptake immediately upon its addition. It was also demon strated that LGF was able to stimulate the A transport system in the plasma membrane, and produced, after 4 - 5 days in culture, a net increase in the amount of DNA of 50%, as well as a 30% increase in the amount of protein with respect to the controls.

In mice, "in vivo", it also produced an increment in the nuclear labeling index, measured by injection of [3H]thymidine and assessed by autoradiography.

The existence of this liver growth factor was likewise detected in plasma of rats injected with two hepatotoxins: thioacetamide and dimethylnitrosamine (Díaz Gil et al., Brit. J. Cancer, 55, 599-604, 1987). Its maximum activity appeared to correspond to the period of maximum hepatic regeneration, disappearing when this process had finalized, for which reason it was assumed that this LGF was associated with the liver regeneration that follows hepatocellular necrosis. However, the composition of LGF continued to be unknown.

DESCRIPTION OF THE INVENTION

It is an object of the present invention to provide a human liver growth factor (h-LGF) constituted by a complex of albumin and bilirubin, for use as a medicine for the treatment of hepatopathies.

Also, the h-LGF is appropiate for use as a diagnosis means and in the follow-up of hepatopathies. According to another aspect of the invention, the h-LGF is used for the preparation of a medicine for the treatment of hepatopathies. To such end, the h-LGF is prepared as an appropiate medicine for the administration of an appropiate dose to a patient.

Brief description of the drawings

- Figure 1 represents HPLC chromatograms of tryptic digestions of (a) performic acid-oxidated LGF and (b) purified serum albumin.
- Figure 2 displays the circular dichroism spectra for LGF and purified serum albumin.
- Figure 3 shows the dose-dependence curve of LGF and albumin-bilirubin preparations "in vivo" (at low doses).
- Figure 4 shows the dose-dependence curve of LGF and albumin-bilirubin preparations "in vivo" (at higher doses).
- Figure 5 represents the percentage of albumin-bound bilirubin necessary to form the liver growth factor.

## Detailed description of the invention

In order to determine the composition of the LGF, a long study was initiated which has led to the establishment of the invention being dealt with in this application. LGF was compared to the albumin of normal rat, purified by the same procedure as that employed with the LGF, in terms of absorbance, fluorescence and circular dichroism spectra, amino acid composition, tryptic maps, antigenic determinants by Ouchterlony plates, electrophoretic mobility, chromatographic behavior with several types of gels and molecular weight by polyacrylamide-gel electrophoresis.

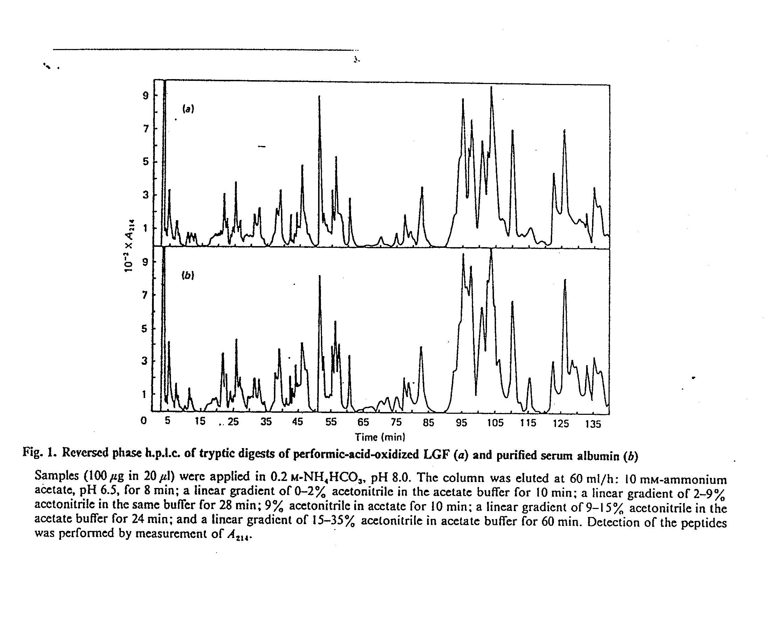

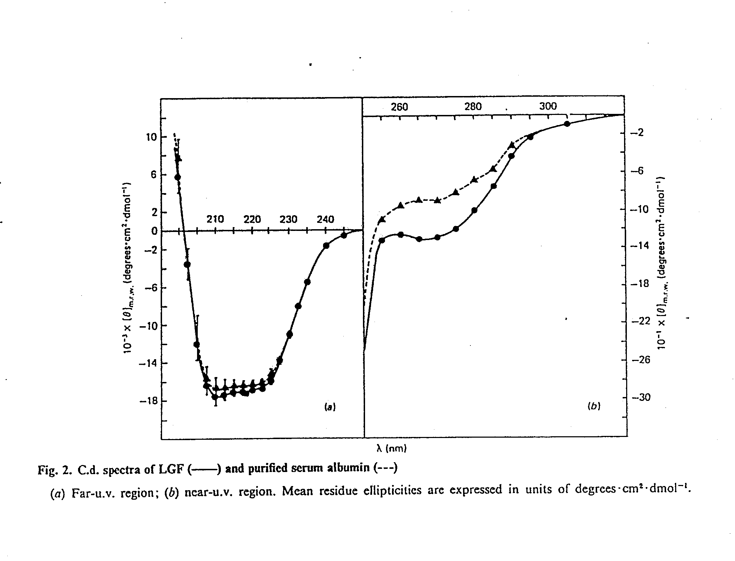

The similarity between the two proteins was observed by comparing their tryptic maps, as can be seen in Figure 1 of the illustrations. The amino acid compositions of the two proteins are very similar (see Table 1). The UV-absorbance spectra of purified serum albumin and LGF coincide. The fluorescence emission spectra for excitation at 295 nm are almost coincident. Only slight differences are observed in the circular dichroism spectra of the two proteins in the near ultraviolet region, although, at the level of the secondary structure, no differences are observed (see Figure 2). The secondary structure of LGF could be composed of 59% alpha-helix, 17% beta-structure and 24% aperiodic conformation, with an average number of 10 residues per helical segment, based on the far-ultraviolet circular dichroism spectrum and using the reference parameters of Cheng et al. (Biochemistry 13, 3350-3359, 1974). The immunodiffusion and immunoelectrophoresis experiments indicated that purified serum albumin and LGF have the same antigenic determinants [a commercial preparation of anti-(rat serum albumin) serum from rabbit (Nordic Immunological Reagents) was used]. All of these results suggest that this liver growth factor contains or is a form of albumin. Therefore, the authors studied the potential effect of both commercial and purified rat serum albumin, F 121(N), as liver growth factors, finding no such activity in these proteins.

Table 1. - Amino acid composition of the liver growth factor; the values for purified rat serum albumin are also included for comparison.

Values are expressed as the nearest integer value based on a $M_r$ of 65000 daltons for both proteins. Three separate determinations were made for each hydrolysis time. Serine and threonine contents were corrected to zero hydrolysis time. Valine and isoleucine contents were determined from 96-h hydrolysates. Hemicystine was determined in the form of cysteic acid after performic acid oxidation of the protein samples at -10°C (Hirs, J. Biol. Chem. 219, 611-621, 1956).

| Amino acids | Composition (residues/M$_r$ 65000) | |
| --- | --- | --- |
| | LGF | Purified albumin |
| Cys | 32 | 31 |
| Asx | 47 | 46 |
| Thr | 33 | 35 |
| Ser | 27 | 28 |
| Glx | 75 | 76 |
| Pro | 29 | 28 |
| Gly | 27 | 23 |
| Ala | 61 | 63 |
| Val | 35 | 36 |
| Met | 6 | 6 |
| Ile | 16 | 15 |
| Leu | 54 | 54 |
| Tyr | 21 | 21 |
| Phe | 27 | 27 |
| His | 16 | 16 |
| Lys | 49 | 49 |
| Arg | 29 | 29 |

In the attempt to explain the activity of LGF as a liver growth factor, one possibility looked at was the existence of a small ligand, X, of very low molecular weight, bound to the albumin in the LGF. Thus it would be the albumin-X complex that presented activity rather than the albumin and the X compound separately. As a further development of this proposal, the hypothetical X ligand might be the true liver growth factor, appearing in situations of partial hepatectomy, but absent in normal rats. The authors of the invention considered the possibility that this X compound could be bilirubin. To test this hypothesis, an experiment was carried out which consisted in incubating normal rat albumin, F 121(N), with increasing quantities of bilirubin. The incubation of the two, bilirubin and albumin, was carried out in 0.1 M phosphate buffer, pH 7.4, for 4 hours in the dark, at room temperature, under the conditions specified by Wooley and Hunter (Arch. Biochem. Biophys. 140, 197-209, 1970). Table 2 shows the activity of the albumin-bilirubin complexes resulting from the incubation of the two components in molecular relationship, r = bilirubin/albumin, variable, and its injection into mice, measuring its effect on DNA synthesis in the liver in the same conditions in which LGF proved to be active (see Biochem. J. 235, 49-55, 1986).

Table 2.

|  |  | Specific incorporation | |
| --- | --- | --- | --- |
|  | n | (dpm/μg of DNA) | (% of control) |
| Control (saline-injected) | 10 | 38.2 ± 16.5 | 100 ± 43 |
| LGF (150 ng/mouse) | 3 | 108.7 ± 11.6 | 286 ± 31[a] |
| F 121(N) (150 mg/mouse) | 3 | 40.5 ± 8.8 | 106 ± 23[b] |
| + bilirubin (r=0.1) | 7 | 47.7 ± 17.3 | 125 ± 45[b] |
| + bilirubin (r=0.5) | 9 | 60.0 ± 16.4 | 157 ± 43[c] |
| + bilirubin (r=0.8) | 15 | 64.0 ± 18.4 | 168 ± 48[d] |
| + bilirubin (r=1.0) | 19 | 101.7 ± 38.0 | 266 ± 99[a] |
| + bilirubin (r=2.0) | 8 | 101.1 ± 32.2 | 265 ± 85[a] |
| + bilirubin (r=3.0) | 8 | 77.9 ± 37.8 | 204 ± 102[c] |
| + bilirubin (r=4.0) | 8 | 73.0 ± 18.7 | 191 ± 49[a] |
| + bilirubin (r=10.0) | 6 | 35.8 ± 18.0 | 94 ± 47[b] |
| Bilirubin (same amount as r=1.0) | 11 | 48.3 ± 18.9 | 127 ± 50[b] |
| Bilirubin (same amount as r=2.0) | 10 | 46.1 ± 14.3 | 121 ± 37[b] |

Dpm, disintegrations per minute; alb, albumin; bil, bilirubin; n, number of mice. [a] $p < 0.001$; [b] no statistical significance; [c] $0.01 < p < 0.02$; [d] $0.001 < p < 0.002$.

From the results in Table 2, the following points should be made:

- Albumin alone or bilirubin alone present no DNA-synthesis stimulating activity in mouse liver. With bilirubin, addi tional injections were carried out at doses ranging from 5 to 214 μg/mouse (ten different doses in all) and in no case was activity detected.

- The incubation of albumin and bilirubin (r = 1 or 2) results in a complex which exactly reproduces the activity of LGF.

- When the bilirubin is incubated in greater or lesser ratios than those mentioned in the preceding point, a lesser activity is detected.

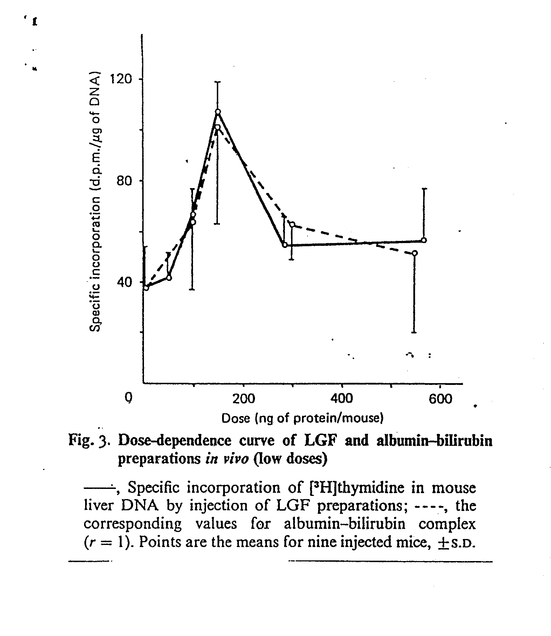

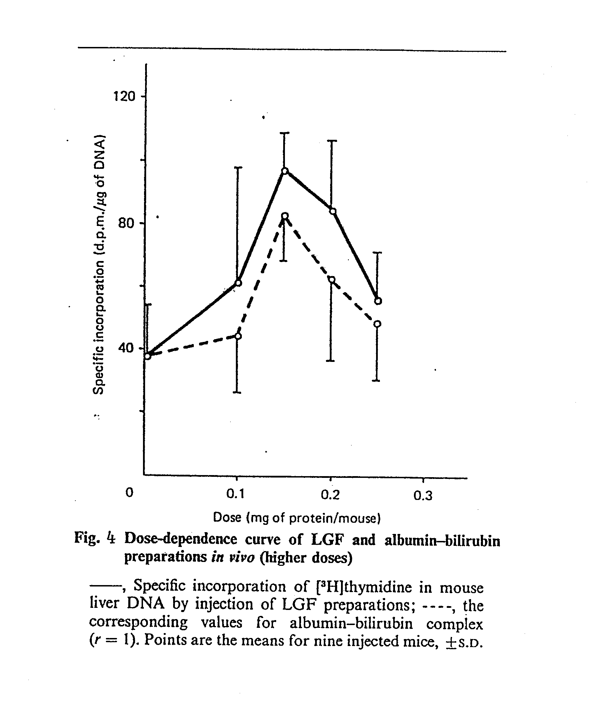

The dose-dependence curve of the albumin-bilirubin complex was compared with that of LGF. F 121(N) plus bilirubin (r = 1) was injected into mouse and the [³H]thymidine uptake was measured in liver DNA. The profile obtained (Figure 3) was identical to that of LGF. When LGF and albumin-bilirubin complex were injected together (150 ng of each protein), the stimulation of DNA synthesis observed was similar to that produced by 300 ng of LGF or bilirubin complex alone (Figure 3). This experiment provides additional evidence for the identity of LGF and an albumin-bilirubin complex. Previous results from the laboratories of the inventors, not presented here, indicated that LGF also exhibits a peak of activity at milligram doses. In order to verify this behavior for the "in vitro"-formed albumin-bilirubin complex (r = 1.0), the experiments illustrated in Figure 4 were performed. The LGF and the albumin-bilirubin complex exert very similar degrees of stimulation.

Finally, the effect of albumin-bilirubin complex (r = 1) on the mitotic index in mouse liver was also measured, comparing it with the corresponding values obtained with LGF. The results are presented in Table 3.

Table 3. Effect of injection of LGF or F 121(N) plus bilirubin (r = 1) on mitotic index in mouse liver

$10^4$ cells were examined microscopically in different fields. (three mice per group). The conditions were those described by Morley and Boyer (Biochem. Biophys. Acta 477, 165-167, 1977).

| Groups of mice | No. of mitoses |
| --- | --- |
| Control (saline-injected) | 2 |
| 150 ng LGF/mouse | 82 |
| 150 ng F 121(N) + bilirubin/mouse | 77 |
| 0.15 mg LGF/mouse | 88 |
| 0.15 mg F 121(N) + bilirubin/mouse | 73 |

In Table 3, it can be observed that the albumin-bilirubin complex mimics the previously described increase in mitotic index provoked by the injection of LGF (Diaz Gil et al. Hepatology 6, 658-661, 1986).

The action of the LGF and the albumin-bilirubin complex in primary rat hepatocyte cultures (stimulation of DNA synthesis) is identical in both cases, as is the concentration of protein at which they exert their maximum stimulatory effect.

All these experiments demonstrated that the LGF is a complex formed by albumin and bilirubin.

For the purpose of determining the amount of bilirubin bound to F 121(N), fluorescence measurements and circular dichroism spectra of the F 121(N) + bilirubin complex formed "in vitro" were assessed for the bilirubin/albumin ratios specified.

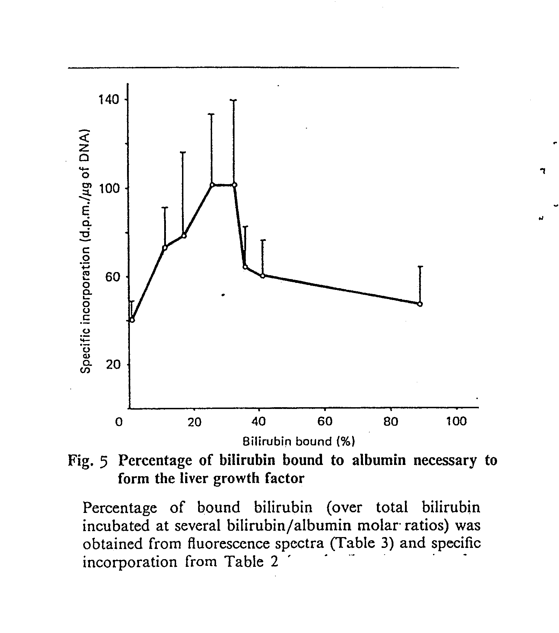

When both ellipticity values at 400 and 452 nm were plotted versus the protein/bilirubin molar ratio, a saturation curve was obtained. However, two inflection points can be observed (results not shown). These factors could be interpreted in terms of two different contributions to the signal of the circular dichroism spectrum arising from bound bilirubin. It is known that albumin possesses two kinds of binding sites for bilirubin, one of high affinity and another of low affinity (Berde et al. J. Biol. Chem. 254, 391-400, 1979; Lavie and Blauer Arch. Biochem. Biophys. 193, 191-203, 1979). Although the percentage of bound bilirubin was estimated from the circular dichroism measurements (Table 4), these data have not been considered in Figure 5. This figure was designed only on the basis of the values obtained from the fluorescence determinations. In fact, the potential existence of two different contributions to the circular dichroism signal upon bilirubin binding could mask the calculation of the value obtained by extrapolation to zero of the parameter (protein/bilirubin)$^{-1}$. The results appear in Table 4.

Table 4. Bilirubin bound to albumin determined by fluorescence and circular dichroism.

The percentage of bound bilirubin was calculated by considering that the fluorescence of bound bilirubin may be estimated by extrapolation to zero in a double-reciprocal plot of the fluorescence increase at 510 nm versus albumin/bilirubin molar concentration. When ellipticity versus albumin/bilirubin double-reciprocal plots are represented, the ellipticity of bound bilirubin can be determined by extrapolation to zero of the value (albumin/bilirubin)$^{-1}$. This value would be that corresponding to the first type of contribution, and can be used to calculate the bound bilirubin.

| Bilirubin/albumin* molar ratio | Bound bilirubin determined by: | | | |
| --- | --- | --- | --- | --- |
| | Fluorescence | | Circular dichroism | |
| | (μmol) | (%) | (μmol) | (%) |
| 0.1 | 0.088 | 88 | NC | NC |
| 0.5 | 0.21 | 42 | NC | NC |
| 0.8 | 0.29 | 36 | NC | NC |
| 1 | 0.33 | 33 | 0.45 | 45 |
| 2 | 0.52 | 26 | 0.60 | 30 |
| 3 | 0.51 | 17 | 0.69 | 23 |
| 4 | 0.44 | 11 | 0.68 | 17 |

* Reference, 1 μmol of albumin; NC, not calculated.

Figure 5 shows the liver growth factor activity of bilirubin-albumin complexes (at different bilirubin/albumin ratios) versus the percentage of bound bilirubin. A plateau of maximal activity is reached and the specific incorporation detected on both sides of the plateau decreases. This result indicates that when bilirubin binds roughly between 26 and 33% with respect to the albumin present, in terms of molar ratios, a maximum activity of the complex is attained.

The demonstration of the chemical structure of LGF was published by the authors of this invention on April 15, 1987, in the Biochemical Journal (243, 443-448, 1987).

The existence, in bile and urine of patients with hemolytic jaundice, of a kind of pigment which undergoes diazotization has been known since 1954 (Biochem. J. 57, 514-518, 1954). Kuenzle et al. (J. Lab. &; Clin. Med. 67, 282-293, 1966) published the quantitative estimate of four fractions of serum which contained bilirubin. One of these fractions was defined as a form of bilirubin firmly adsorbed to serum albumin, which is denominated the δ fraction or biliprotein. Since then, the biological role of the biliprotein had remained completely unknown.

Continuing the investigation, the inventors studied the possible identity of the LGF with the biliprotein. To do this, rats were hepatectomized, to be sacrificed at different times (0, 6, 12, 18, 24, 36 and 48 hours). In the plasma resulting in each case (two rats per group), the concentration of bilirubin was determined by the procedure of Seligson et al. (Clin. Chem. 31, 1317-1321, 1985) and the LGF was purified, measuring its activity as a stimulator of the DNA synthesis in mouse liver. The fourteen points obtained for biliprotein and LGF activity were represented in a graph, resulting in a straight line with a correlation coefficient of 0.999. Thus, it was shown that the LGF is the complex denominated biliprotein or δ bilirubin (Díaz Gil et al. Hepatology, in press, 1988).

Parallelly, the inventors published (Hepatology 6, 658-661, 1986) the detection of a DNA-synthesis promoter activity in plasma of patients with acute hepatitis B. Shortly after, this activity was purified in the form of h-LGF ("h" indicating its human origin; this entity was initially designated HP, as in rat). The characteristics were apparently identical to those of the rat LGF (Hepatology, 6, 769, Abstract 2, 1986). This h-LGF also showed DNA-synthesis stimulator activity in primary human hepatocyte culture.

Later, a study was performed of the chemical characteristics of h-LGF in comparison with the purified plasma albumin of normal individuals, without known liver pathology, consisting of the same assays which were indicated above for LGF and rat albumin. The conclusion was the same: albumin is a constituent element of LGF. Moreover, when human albumin is incubated with bilirubin, the resulting complex produces exactly the same activity as h-LGF with respect to:

- "in vivo" DNA synthesis in mouse liver
- increase in mitotic index of mouse hepatocytes "in vivo"
- DNA synthesis in primary rat hepatocyte culture, etc.

Like r-LGF (liver growth factor from plasma of partially hepatectomized rat), h-LGF then is an albumin-bilirubin complex.

In relation to all this, several authors have communicated in recent years the existence of biliprotein in human serum in pathologies with hepatobiliary involvement (Clin. Res. 496A, 1982; Clin. Chem. 28, 1579, 1982; N. Engl. J. Med. 309, 147-150, 1983). This finding has been underlined by the fact that biliprotein is not detected in normal individuals without liver pathology; nor is it found in the hyperbilirubinemia of newborns, patients with Gilbert's disease or cases of hemolytic anemia. It appears, therefore, that biliprotein is detectable only in hepatobiliary disorders, fact which clearly coincides with the detection of LGF, in human and animal models, in adults as well as in newborns and children (cytomegalovirus infection and biliary atresia).

The authors of the invention corresponding to this application likewise showed, in serum of patients with hepatitis B, the identity of h-LGF with biliprotein. To do this, they purified the h-LGF from samples of patient serum and injected it into mice, measuring the stimulation of DNA synthesis in liver and, therefore, the activity of h-LGF. In the same serum samples, the amount of biliprotein was measured by HPLC. Both values, when represented graphically, result in a straight line with a correlation coefficient of 0.999 (n = 9).

The number of albumin molecules which make up the albumin-bilirubin complex (h-LGF or biliprtoein) varies greatly. In patients with acute hepatitis B, in the initial days after diagnosis, it is usually 10% (3.9 - 4.4 ng/ml serum), although this value varies according to the patient's recovery and the presence of additional infections such as delta virus, in which values of up to 8.0 ng/ml serum or more may be detected.

Example 1

Plasma was taken from 5 patients with viral hepatitis B and h-LGF was isolated and purified. The samples were taken in the initial days after diagnosis of the disease. The purification technique for h-LGF and the measurement of its activity by injection into mice is described in the work of Díaz Gil et al. (Biochem. J. 235, 49-55, 1986; Hepatology 6, 769, Abstract 2, 1986). A unit of activity (UA) is defined as the amount of h-LGF/mouse necessary to reach maximum uptake of [³H]thymidine into mouse liver DNA (roughly 100 dpm/μg DNA).

Naturally, the purer the preparation of h-LGF the smaller the amount required to reach maximum [³H]thymidine uptake into mouse liver; moreover, it will have greater activity (in UA/mg protein). On the other hand, the greater the impurity of the h-LGF preparation, the larger the amount of protein to be injected, while its activity in UA/mg protein will be lesser. Here the terms "purer" or "less pure", when applied to a h-LGF preparation, should be understood to refer to a higher or lower percentage with respect to the albumin in the albumin-bilirubin complex, which exhibits the liver growth factor activity.

The mean h-LGF activity measured in the five cases of acute hepatitis B was 7138 ± 921 UA/mg protein. This value diminished as the patients' progressed favorably. Taking into account that the h-LGF activity is not detected in normal individuals without hepatic pathology, the measurement of h-LGF throughout the course of hepatitis is a parameter for the follow-up and diagnosis of this disease, indicating a true index of liver regeneration, which at present is not available.

Likewise, h-LGF activity has been measured in three cases of fulminant hepatitis leading to the death of the patients. The mean of this activity was 650 ± 70 UA/mg protein, representing approximately 9% of that found in acute cases. In another two cases of fulminant hepatitis which did not result in the death of the patients, the mean value was 6950 ± 820, suggesting that the determination of h-LGF constitutes a diagnostic index of fulminant hepatitis, entity which to date has not been available.

Example 2

A total of ten patients with acute hepatitis B were studied (AST and ALT values greater than 200 IU/l and presence of HBsAg, HBcAg and IgM-bound anti-HBc). Five of those studied were cured, while the other five had torpid progress, probably leading to chronicity.

The determination of the liver growth factor, h-LGF, was first performed in each patient after the diagnosis of the disease and at 10-day intervals until hospital discharge, which occurred 30 - 40 days after diagnosis. In every case, an additional determination was performed 1 year later.

The level of h-LGF in the initial period of maximum clinical activity was very elevated 7138 ± 921 UA h-LGF/mg protein), and diminished parallelly to the remission of the clinical picture, reaching 200 UA h-LGF/mg protein at the time of discharge. This coincided with an analogous decrease in the transaminase and bilirubin levels.

One year later, the group of patients that presented no clinical or biochemical activity showed no h-LGF activity either. In contrast, those patients with residual activity at that time also presented h-LGF activity, with values of 200 -500 h-LGF/mg protein.

From these data, it could be deduced that the measurement of h-LGF is a follow-up index for liver regeneration, which to date has not been available, and that its residual activity one year after hospital release could be related to presence of the disease.

Example 3

The variations of h-LGF have been studied in serum of seven patients after liver transplantation. The determination of h-LGF was done by HPLC.

The patients subjected to the study were between 27 and 52 years of age, of both sexes, and their pre-transplantation diagnoses were: cirrhosis of diverse etiology (5 cases), primary sclerosing cholangitis (one case) and hepatic traumatism (one case).

After liver transplantation, the variations in serum bilirubin, transaminases, GGT, alkaline phosphatase and bile acids were assessed, as well as other determinations. The follow-up of all these parameters was used to compare the elevations of each with those corresponding to h-LGF. The following facts are worthy of note:

a) h-LGF is the most sensitive parameter of all those studied in situations of graft damage (it reflected the highest number of variations, 22, versus the other parameters which ranged from 8 to 20).

b) Rises in transaminases are always accompanied by elevations of h-LGF, probably in relation to the cytolysis-liver regeneration.

c) On three occasions, the only alteration detectable was that of h-LGF, probably due to minimal liver damage and subsequent regeneration, not detectable by other methods.

d) In some cases of cholestasis with slight or no alteration in the transaminases, there was rise in h-LGF.

From these data, it could be deduced that the measurement of h-LGF is a good index of liver damage after transplantation and could be used in the follow-up of individuals who have undergone this operation.

**Claims**

1. Human liver growth factor (h-LGF) constituted by a complex of albumin and bilirubin, for use as a medicine for the treatment of hepatopathies.

2. Human liver growth factor (h-LGF) constituted by a complex of albumin and bilirubin, for use as a diagnosis means and in the follow-up of hepatopathies.

3. Use of the human liver growth factor (h-LGF) for the preparation of a medicine for the treatment of hepatopathies, characterized in that the h-LGF defined in claim 1 is prepared as an appropiate medicine for the administration of an appropiate dose to a patient.

0287493

**Fig. 1. Reversed phase h.p.l.c. of tryptic digests of performic-acid-oxidized LGF (a) and purified serum albumin (b)**

Samples (100 µg in 20 µl) were applied in 0.2 M-NH₄HCO₃, pH 8.0. The column was eluted at 60 ml/h: 10 mM-ammonium acetate, pH 6.5, for 8 min; a linear gradient of 0–2% acetonitrile in the acetate buffer for 10 min; a linear gradient of 2–9% acetonitrile in the same buffer for 28 min; 9% acetonitrile in acetate for 10 min; a linear gradient of 9–15% acetonitrile in the acetate buffer for 24 min; and a linear gradient of 15–35% acetonitrile in acetate buffer for 60 min. Detection of the peptides was performed by measurement of $A_{214}$.

Fig. 2. C.d. spectra of LGF (——) and purified serum albumin (---)

(a) Far-u.v. region; (b) near-u.v. region. Mean residue ellipticities are expressed in units of degrees·cm²·dmol⁻¹.

**Fig. 3. Dose-dependence curve of LGF and albumin–bilirubin preparations *in vivo* (low doses)**

———, Specific incorporation of [³H]thymidine in mouse liver DNA by injection of LGF preparations; ----, the corresponding values for albumin–bilirubin complex ($r = 1$). Points are the means for nine injected mice, ±s.D.

0287493

Fig. 4 **Dose-dependence curve of LGF and albumin–bilirubin preparations** *in vivo* **(higher doses)**

——, Specific incorporation of [³H]thymidine in mouse liver DNA by injection of LGF preparations; - - - -, the corresponding values for albumin–bilirubin complex ($r = 1$). Points are the means for nine injected mice, $\pm$s.d.

**Fig. 5 Percentage of bilirubin bound to albumin necessary to form the liver growth factor**

Percentage of bound bilirubin (over total bilirubin incubated at several bilirubin/albumin molar ratios) was obtained from fluorescence spectra (Table 3) and specific incorporation from Table 2